(19) Europäisches Patentamt
European Patent Office
Office européen des brevets

(11) Publication number: **0 226 234 B1**

(12) **EUROPEAN PATENT SPECIFICATION**

(45) Date of publication of the patent specification: **08.08.90**

(51) Int. Cl.⁵: **B01J 37/16**, B01J 23/66, C07D 301/10, C07C 45/38

(21) Application number: **86201918.9**

(22) Date of filing: **31.10.86**

(54) **A process for preparing a silver-on-carrier catalyst.**

(30) Priority: **01.11.85 NL 8502991**

(43) Date of publication of application: **24.06.87 Bulletin 87/26**

(45) Publication of the grant of the patent: **08.08.90 Bulletin 90/32**

(84) Designated Contracting States: **BE DE ES FR GB IT NL SE**

(56) References cited:
**EP-A- 0 091 165**

**JOURNAL OF CATALYSIS, vol. 61, 1980, pages 348-358, Academic Press, Inc.; G.C. BOND et al.: "Noble metals supported on Phillips-type catalysts: preparation, characterization, and activity for ethane hydrogenolysis"**

(73) Proprietor: **THE DOW CHEMICAL COMPANY, 2030 Dow Center Abbott Road P.O. Box 1967, Midland Michigan 48640-1967(US)**

(72) Inventor: **Geus, John Wilhelm, Gezichtslaan 100, NL-3723 GJ Bilthoven(NL)**
Inventor: **Melma, Garmt Ricardo, Aalberselaan 4c, NL-3818 XM Amersfoort(NL)**

(74) Representative: **Smulders, Theodorus A.H.J., Ir. et al, Vereenigde Octrooibureaux Nieuwe Parklaan 107, NL-2587 BP 's-Gravenhage(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

This invention relates to a process for preparing a silver catalyst on an inert carrier material, which comprises precipitating silver ions from a solution by reduction to metallic silver in the presence of an inert carrier material.

A similar process is disclosed in European Patent application 91165. In that process the starting product is a thermostable inert carrier, for example, aluminium oxide or silicon dioxide to which an element or compound has been applied, in very finely-divided form, which has to perform two functions. The first is the catalytic acceleration of the reduction of dissolved silver ions to metallic silver with a gaseous or dissolved reducing agent. As example of such reducing agents are mentioned hydrogen, formaldehyde or glucose.

The second function is the anchoring of the silver particles thus applied to the carrier to the carrier surface. This anchoring of the silver particles to the carrier surface prevents the silver particles from going to move over the carrier surface; the contact of silver particles moving over the carrier surface leads to rapid coalescence of the particles, as a consequence of which the silver area decreases markedly.

The use of this prior process with powdered carrier materials suspended in the silver ions containing solution leads to excellent results. It is less suitable, however, for loading large carrier bodies with silver particles. In reactors in which the catalyst is present in a solid bed, powdered catalysts cannot be used, because they would lead to an intolerably high pressure drop. As reactors with a solid catalyst bed are frequently used, solid catalyst will often have to be used as larger bodies, for example, cylinders or rings in sizes of, for example, 5 to 10 mm. Breakage of catalyst bodies in the reactor leads to increased pressure drop, and poor distribution of the reactants over the catalyst bed. The catalyst bodies must therefore have a high mechanical strength. The catalyst pellets must be able to resist the often large forces exerted on them as the reactor is being filled, and during the course of the reaction. They must also be resistant to the thermal shocks which occur as the reactor is being put into or out of operation. The mechanical strength is therefore one of the most important properties of catalyst bodies.

Powdered carries laden with (a precursor of) an active component often readily permit being processed to bodies of the required mechanical strength. In some cases, however, it is of advantage to start from carrier material previously processed to larger bodies, e.g., cylinders having a diameter of 5 mm and a length of 10 mm. This is effected, for example, by pelletizing or extruding the carrier material. For the production of spherical bodies with a diameter of, for example, 5 mm, the sol-gel method is very suitable. Only after the formation to larger bodies is the (precursor of the) active component applied.

In the application of silver particles to larger carrier bodies by the above prior process, one must proceed with great care. To obtain a homogeneous distribution of the silver particles throughout the entire surface of the carrier, the rate of the reduction reaction and of the transport of silver ions and the molecules of the reducing agent to the interior surface of the catalyst bodies must be attuned to each other. The rate of the reduction reaction is commonly adjusted by a suitable selection of the temperature. In addition to the sizes of the carrier bodies, the diameter and the length of the pores in the carrier must be taken into account. Generally speaking, the desired homogeneous distribution of the silver particles over the surface of the carrier is only achieved if the rate of transport is higher than the rate of the reduction reaction. On a technical scale, therefore, the loading of larger bodies of the carrier material is not a simple matter, partly because keeping a considerable quantity of the carrier material in suspension in a larger volume of liquid is diffcult and often leads to abrasion of the carrier bodies.

Especially in the case of silver catalyst, there is a great need for a process for applying the silver homogeneouly over the interior surface of previously shaped carrier bodies. As silver catalyst are often used for the selective oxidation of organic compounds, the carrier used is preferably as inert as possible, Alpha-aluminium oxide has a highly inert surface and is therefore excellently suitable for the purpose. Processing powdered, silver-coated alpha-aluminium oxide to mechanical strength, however, is difficult. Generally speaking, hydroxides can be well processed to mechanically strong bodies by pressing or extrusion. Probably, the formation of hydrogen bridges plays an important role in this connection. Thus aluminium oxide still containing hydroxyl groups readily permits being processed to catalyst bodies of the desired strength. In alpha-aluminium oxide, however, hydroxyl groups are virtually completely absent, as a result of which a powder of this material is poorly processed to the desired bodies. Carrier bodies of alpha-aluminium oxide are therefore mostly made by starting from gamma-aluminium oxide. This material contains a larger number of hydroxyl groups and, as stated before, can accordingly be properly processed to mechanically strong bodies. After the formation to carrier bodies the material is kept at such a high temperature that the conversion to the stable alpha-aluminium oxide proceeds. During this conversion, the shape of the bodies is retained, and their mechanical strength is often considerably increased. In the case of alpha-aluminium oxide, therefore, one can actually only start from carrier bodies that have been previously produced. Processing alpha-aluminium oxide powder loaded with silver particles is technically difficult.

In the process according to the above European patent application 91165, the dissolved, and if necessary complexed, silver ions and the molecules of the reducing agent must migrate to a position on the carrier surface where the compound which accelerates the reduction is present As a consequence, the rate at which the silver particles formed on the carrier grow partly depends on the rate at which

the silver ions and the molecules of the reducing agent can diffuse to the silver nuclei. Small particles of the carrier material contain relatively short pores. When these pores are not too narrow, the rate of transport is generally high relatively to the rate of (chemical) reduction. In that case the silver nuclei will grow uniformly. If, however, the accessibility to certain parts of the interior surface of the carrier bodies is less, the rate of transport will play a role. The silver nuclei will then grow non-uniformly.

Accordingly, the application of silver particles to somewhat larger bodies of alpha-aluminium oxide (which hence contain longer pores) can be effected satisfactorily by the known process, but yet continue to remain difficult to perform when carried out on technical scale. Certainly for silver catalysts, which are often applied to alpha-alumina as a carrier, an improvement of the above process is therefore attractive.

It is accordingly an object of an invention to provide a process in which silver particles can be uniformly applied over the (interior) surface of larger carrier bodies in a technically simple manner.

It has been found that the preparation of a silver-laden carrier can be carried out in a very simple manner without the use of a separately added dissolved or gaseous reducing agent, if the starting product is an inert carrier material to which previously an oxidizable metal or an oxidizable compound has been applied.

In the process according to the invention, the reduction of the dissolved silver ions is essentially effected by a reducing agent previously applied to the carrier as an oxidizable metal and/or as an oxidizable solid compound. Accordingly, the reduction takes place not by a dissolved reducing agent, but by a reducing agent solely present on the surface of the carrier. Reduction is the liquid phase, where no reducing agent is present, need not be prevented, therefore. As the metallic silver formed is insoluble, the distribution of the silver over the carrier surface will correspond to that of the previously applied reducing agent. If the reducing agent is selected so that, also after oxidation, it can perform an anchoring function, excellent adherence of the silver to the carrier surface is also ensured, because in fact the silver particles are exclusively formed on the anchoring compound.

In the case of porous particles whose pores are filled with liquid, the rate of physical transport already get an appreciable influence with particles sizes above 10 µm with a pore diameter of about 10 nm. In these cases, the rate of the chemical reaction must be kept low relative to the rate of transport by way of the temperature and/or concentration. In the process according to the invention, the ratio of the rate of transport and the reduction rate does not play any role. The reduction proceeds in the last stage of the process exclusively at the sites where the solid reducing agent has not as yet reacted. If this solid reducing agent has previously been applied homogeneously over the surface of the carrier, the silver will also be uniformly deposited over the carried surface.

Accordingly, using the process according to the invention, silver is uniformly applied to an inert thermostable carrier and in finely-divided form via an anchoring element or an achoring compound by previously loading the carrier with a metal or a compound capable of reducing silver ions, and capable of binding silver particles on the carrier surface. Examples of compounds and elements capable of adhering silver particles to carrier materials are metals or metal oxides, such as lead, lead oxide, bismuth and bismuth oxide, and tin oxide. Furthermore, rhenium or a metal selected from the second or third period of the group VIII metals of the Periodic Table, or an alloy of these metals.

It has been found that metals such as platinum and palladium and oxides such as tin oxide and lead oxide effect an excellent adherence of silver to the carrier material. The selection of elements or compounds capable of reducing silver ions can be readily effected via the electrochemical potentials.

Suitable reducing and adhering compounds are in the first place compounds of metal ions having more than one stable valence, such as tin, lead, manganese, arsenic, antimony and the like. The oxides are present on the carrier in the lower-valent form, whereafter reaction with silver ions leads to the higher-valent form which also anchors the metallic silver formed. Thus tin, lead and manganese are applied to the carrier in the bivalent state, and arsenic and antimony in the trivalent state. The reduction of the silver can also be performed with metals, which thereby pass into adhering oxides. Thus metallic tin or lead can be used for the reduction. It is of importance that the adhering element or the adhering compound should not detract from the selectivity of the silver catalyst ultimately obtained.

There are two ways of applying the (hydr)oxide to the carrier material in the reduced form. In accordance with a first method, the lower-valency oxide as such is applied to the carrier. After the application of the reducing agent oxidation by atmospheric oxygen must be prevented. When thereafter a silver ions containing solution is added, reduction to insoluble metallic silver proceeds on the carrier surface. In accordance with a second method, the component is primarily applied to the carrier in the oxidized state. In a separate step the compound present on the carrier is reduced; in this case reduction can be effected to a lower-valency oxide, but also to the corresponding metal. In the subsequent process step this metal then reacts with the silver. The reduction to the metal should be effected with due care; in certain cases reduction to the metal leads to marked sintering of the metal. The desired fine distribution of the silver by local reduction by the metal can then not be realized.

It is noted that Journal Catalysis Vol. 61 (1980) 348-358 describes the reaction of chromium(II)ions applied to the surface of silicon dioxide with, for example, silver perchlorate. This, however, only resulted in reduction of the anion bonded to silver; metallic silver was not formed.

The reducing element or the reducing compound can be applied to the carrier in a manner known to those skilled in the art. The method used must lead

to a uniform distribution of this reducing agent over the surface of the carrier. A suitable method is deposition-precipitation, a process especially directed towards obtaining a uniform distribution of an active precursor over the surface of the carrier (J.W.Geus, in "Preparation of Catalysts" III, G.Poncelet, P.Grange and P.A.Jacobs, editors, pp. 1-33, Elseviers, Amsterdam 1983). For pre-shaped carrier bodies, the injection process of this method is generally less satisfactory. Preferably, the carrier bodies are impregnated with a solution of an active precursor together with an agent which when the temperature is increased leads to precipitation of the precursor. One example is impregnation with a urea-containing solution; when the temperature is increased the hydrolysis of the urea increases the pH and, precipitation takes place. An "incipient wetness" impregnation is here attractive. In this case just sufficient solution is added to completely fill the carrier pores. Because, in these cases, it is possible to use highly concentrated solutions, the desired degree of loading with the reducing agent can yet be achieved.

Another, highly suitable procedure for loading pre-shaped carrier bodies with a precursor of an active component or of a reducing agent is also based on "incipient wetness" impregnation. In that case the pore system of the carrier is filled fully, or substantially fully, with a soluble complex of (a precursor of) the reducing agent. The complex is so selected that no nucleation of crystalline compounds occurs and that the viscosity of the solution increases when the solvent is evaporated by heating. After thermal decomposition of the complex (the precursor of) the reducing agent is deposited on the surface of the carrier in uniformly distributed form.

The choice of the most suitable complexing compound depends on all sorts of factors, such as the element to be complexed and the valence thereof, the temperature at which it is desired to carry out the thermal treatment, and the carrier material to be impregnated. Suitable complexing agents are, for example, EDTA, citrates, lactates, and the like. Mostly, heating in an oxidizing medium is required to remove the last residues of the complexing agent by oxidation. The compound deposited on the carrier must then generally be reduced, as described above, in a separate reaction step.

The reduction can be carried out with hydrogen or carbon monoxide. By a suitable selection of the temperature and the hydrogen/water or carbon monoxide/carbon dioxide ratio the degree of reduction can be controlled. By these means the reduction to a lower-valency oxide or to the corresponding metal can be accomplished as desired. As observed above, in the case of reduction to the metal, care must be taken that the metal does not begin to sinter on the carrier. When metals are susceptible to sintering, sintering can be prevented by incomplete reduction and by keeping the laden carrier at a high temperature for a short period of time only. In addition to the above gaseous reducing agents, dissolved reducing agents, such as formaldehyde, can be used for carrying out reduction. This latter is attractive if the desired quantity of silver cannot be deposited in one reaction step. When the reducing agent present on the carrier has been completely used, more silver can be deposited on the carrier by again reducing the oxidized agent. Because, in the case of a liquid-phase reduction the intermediate separation of the partially laden carrier from the liquid and drying are not necessary, this is attractive.

In the process according to the invention, the use of compounds of tin or of metallic tin is highly attractive. Tin oxide is known to anchor silver excellently on a carrier surface. Moreover, tin is stable as tin(IV), tin(II) and as a metal, while it can be readily applied to a carrier in the desired uniform, finely-divided manner. For example, tetravalent tin can be applied to a carrier and subsequently reduced with hydrogen to form bivalent tin or metallic tin. In a next step, the tin(II) or the tin(0) is reacted with silver ions. Tin(IV) can also be reduced with formaldehyde in the liquid phase at low temperatures. As the reduction can be readily carried out several times, tin is excellently suitable to realize a relatively high silver load. An additional advantage is that the electrochemical potentials for the oxidation of tin(0) and tin(II) by silver ions are in a very favourable range.

The reduction of the dissolved silver compounds by the reducing agent present on the carrier material is usually best effected in an aqueous medium. The silver compound is dissolved therein and the carrier laden with the reducing agent in suspended in it. The term "aqueous medium" as used herein means water (including aqueous liquid such as alkaline or acid aqueous salt solutions) or a solution of water with a water-miscible liquid, such as a lower alcohol, for example methanol or ethanol, a lower ketone, for example, acetone, or other analogous compounds. It is also possible to use organic liquids in which a suitable silver-containing compound is soluble.

Suitable silver compounds are known silver salts and silver complexes, such as silver nitrate and the silver-ammonia complex. Unlike the process as disclosed in European patent application 91165, the choice of the silver compound is not critical in the process of the present invention. The only factor that must be taken into account is the electrochemical potential of the silver in the compound used.

The most suitable conditions for reducing the silver depend on a plurality of factors, including the reducing agent applied to the carrier and the dissolved silver compound used. The properties of the carrier, the desired properties of the resulting catalyst and the temperature of the suspension in which the reduction is carried out also play a role. Although the temperature at which the reduction is carried out depends on the reducing agent and the silver compound to be reduced, it is generally possible to use temperatures up to the boiling point of the liquid in which the carrier laden with the reducing agent is suspended.

The reduction of the silver is continued until the desired quantity of metallic silver has been deposited on the carrier. Mostly, however, the reduction proceeds instantaneously. If it is desired to apply silver to relatively large carrier bodies with narrow

pores, the slow transport may limit the rate of the reduction of the silver. In that case an ion-selective silver electrode is valuable. By using an ion-selective silver electrode, the silver ions concentration in the solution can be followed easily and continuously. From the reduction of the concentration of dissolved silver ions, the loading of the carrier with silver can be directly calculated. In order to prevent re-oxidation of the finely-divided metallic silver deposited, the reduction is preferably carried out in an oxygen-free atmosphere. This is also required to prevent the oxidation of the reducing agent present on the carrier.

The desired quantity of silver to be deposited depends on the reaction for which the catalyst will be used. Sometimes, a relatively low degree of loading with small, thermostable silver particles will be sufficient to realize a selective oxidation. In other cases a high degree of loading with silver is desirable.

In order to achieve a high degree of loading, just sufficient silver ions may be added to react with the reducing agent present on the carrier. After the silver has been deposited on the carrier, the reducing agent is reduced a second time, preferably in the liquid phase. Thereafter, the quantity of silver capable of reacting with the reducing agent is added again. Excess reducing agent is removed, for example, by filtration, and a next dose of silver ions is reacted. This procedure can be repeated a number of times.

After the desired quantity of silver has been deposited on the carrier, the carrier thus laden is separated from the liquid reaction medium and dried at elevated temperature, for example, at 50 to 200°C. The separation from the liquid is preferably effected under oxygen-free conditions. The oxidation of the often extremely small silver particles can lead to redissolution in the liquid. When the catalyst has been separated from the liquid, the material can be kept in the air without any objections.

The catalysts produced in this manner can be used as such. It is also possible for the catalysts to be previously subjected to an oxidation treatment at elevated temperature, or for it to be previously reduced. It has been found that a thermal treatment at high temperature does not lead to appreciable sintering of the silver particles.

The silver catalysts according to the invention exhibit an exeptional thermal stability and are suitable for use as a catalyst in a large number of industrial catalytic reactions, such as oxidative dehydrogenation, dehydrocyclisation and (selective) oxidation. Catalysts prepared in accordance with the invention are extremely suitable for the production of ethylene oxide from ethylene and of formaldehyde from methanol.

Whereas the above uses of the catalysts according to the invention relate to known reactions which can be carried out more economically with the catalyst according to the invention, the high thermostability of the catalysts also opens the way towards new processes which do not as yet exist.

The invention is illustrated in and by the following examples.

## EXAMPLE I

Preparation of a silver/tin oxide on alpha-alumina carrier.

In this example, a powdered carrier is used. This is done to show that both the tin and the silver can be applied to a suspended carrier with good homogeneity by the process according to the invention. The laden powder can be investigated in the scanning electron microscope without mechanical treatment. Examination of the transmission electron microscope also requires only minor mechanical treatment of the powder. This catalyst serves as a model for an ethylene oxide catalyst. The starting product, therefore, is a low-area carrier, to which a small quantity of tin oxide and silver is applied.

For this catalyst and alpha-alumina carrier of Fluka was used (Fluka A.G. CH-9470 Buchs, "purum" 0.8 $m^2g^{-1}$).

In 80 ml water, 10.0 g sodium hydroxide was dissolved and subsequently, carefully, 0.69 tin(II) acetate. The tin(II) acetate must be dissolved slowly and with care to avoid the formation of the poorly soluble tin(II) oxide. Furthermore, tin(II) oxide is known to disproportionate into tin(0) and tin(IV) oxide in concentrated lye.

The stannite solution thus prepared was subsequently introduced into a precipitation vessel and made up with boiled, deionized water to a total volume of 150 ml. Boiling to remove dissolved oxygen is of great importance; otherwise tin(IV) is formed, and the reduction of the silver is less complete. The solution was kept free from oxygen by passing a nitrogen stream through the solution. The ph of the solution was 12.2. In this solution 30.0 g of the above alpha-alumina was suspended.

With vigorous stirring a solution consisting of 25 ml concentrated nitric acid and 200 ml deionized (boiled and hence oxygen-free) water was then slowly injected into the suspension below the surface of the liquid over a period of about 1.5 hours until the pH had decreased to 7.0. This caused the tin(II) to be precipitated on the carrier as hydrated tin(II) oxide.

After the resulting suspension has been allowed to stabilize for about 15 minutes, a solution of 0.50 g silver nitrate in 100 ml deionized water was injected under the surface of the suspension in approximately 30 minutes. The reduction of the silver ions to metallic silver by the tin(II) proceeded virtually instantaneously. This was apparent from the suspension turning black and from the reduction of the pH to a final value of 4.7 by the following reaction:

$$\text{"}SnO.xH_2O\text{"} + H_2O + 2\,Ag^+ \longrightarrow SnO_2 + 2\,H^+ + 2\,Ag(O)$$

The suspension was filtered and washed with water and finally dried at about 120°C for about 20 hours. The washing water contained little silver. This could be demonstrated with hydrochloric acid. A minor loss was shown in that the silver content calculated was 1.0 by weight, whereas a silver content of 0.74% by weight was measured. The tin content was also somewhat lower, namely, calc. 1.1 % by weight and measured 1.0% by weight.

Elemental analysis in the scanning electron microscope showed that the silver was present in the catalyst together with the tin. Furthermore, the carrier was shown to be uniformly covered with the silver/tin oxide particles precipitated. In the transmission electron microscope the alumina particles turned out to be covered with finely-divided material. According to the electron diffraction pattern, large portions of the carrier surface were covered with poorly crystallized tin(III) oxide and metallic silver. In addition, larger silver particles occurred, which exhibited a sharp electron diffraction pattern.

EXAMPLE II

Preparation of a silver/tin oxide catalyst on preformed $Al_2O_3$.

In this case the starting product was cylindrical alumina pellets with a diamter of about 5 mm and a length of about 7 mm. In this case, too, aluminium oxide with a low specific area was used, as a result of which a catalyst was obtained, suitable for selective oxidations of organic chemical compounds.

3.0 g tin(II) oxalate and 4.3 g EDTA were mixed with 25 ml water. To this, about 8 ml concentrated ammonia (25%) was added until a pH value was reached of 9.0. The resulting solution was just still clear.

40.0 g alumina pellets were evacuated, whereafter the above solution was impregnated into the pellets. The liquid was fully absorbed by the pellets ("incipient wetness"). Thereafter the water was evaporated by heating the pellets in vacuo at 80°C.

After the pellets had cooled, they were impregnated with a solution of 3.52 g silver nitrate in about 30 ml water. This was also effected by the "incipient wetness" method, whereby the liquid was fully absorbed by the pellets. Black discoloration of the pellets showed that the reduction of the silver proceeded instantaneously. The catalyst was finally dried at 120°C for about 20 hours.

The catalyst thus obtained still contains organic material from the EDTA. This can be removed by oxidation at elevated temperature. A suitable temperature for this purpose is 250°C. The silver is not appreciably sintered by this treatment.

In the scanning electron microscope, the distribution of the tin and the silver of the surface of a cut pellet was measured by means of energy-dispersive analysis of emitted X-ray photons. These two elements were found to be uniformly distributed over the cleavage face of the pellet. Powdered material was investigated in the transmission electron microscope. Here again, poorly crystallized, finely-divided tin(II) oxide was found with small silver particles. In addition some larger silver particles occurred.

**Claims**

1. A process for preparing a silver catalyst on an inert carrier material, which comprises precipitating silver ions from a solution by reduction to metallic silver in the presence of an inert carrier material, characterized in that the reduction is mainly effected by means of a reducing agent for the silver ions, which is present on the surface of the inert carrier material in the form of an oxidizable metal and/or metal compound.

2. A process as claimed in claim 1, characterized by using as the inert carrier material a shaped carrier body.

3. A process as claimed in claims 1–2, characterized in that the oxidizable metal and/or the metal compound is selected from the group of tin, lead, manganese, arsenic, antimony, and compounds thereof.

4. A process as claimed in claim 3, characterized by using an Sn(II)-compound.

5. A process as claimed in claim 4, characterized by using optionally hydrated SnO.

6. A process as claimed in claims 1–2, characterized in that the carrier material on which the oxidizable metal or the oxidizable metal compound is present has been produced by reduction of a reducible metal compound applied to the carrier material.

7. A process as claimed in claim 6, characterized by using as the reducible metal compound an Sn(IV)-compound.

8. A process as claimed in claim 7, characterized by using as the carrier material calcined $SiO_2$ and/or $Al_2O_3$.

9. A process as claimed in claims 1–2, characterized by using as the carrier material calcined $SiO_2$ and/or $Al_2O_3$.

10. A process as claimed in claim 9, characterized by using $\alpha$-$Al_2O_3$.

11. A process as claimed in claims 1–2, characterized in that the reduction of the silver ions is carried out in a substantially oxygen-free atmosphere.

**Patentansprüche**

1. Verfahren zum Herstellen eines Silberkatalysators auf einem inerten Trägermaterial durch Abscheiden von Silberionen aus einer Lösung durch Reduktion zu metallischem Silber in Gegenwart eines inerten Trägermaterials, dadurch gekennzeichnet, daß die Reduktion hauptsächlich durch ein Reduziermittel für Silberionen erfolgt, das auf der Oberfläche des inerten Trägermaterials in Form eines oxidierbaren Metalles und/oder einer Metallverbindung vorhanden ist.

2. Verfahren nach Anspruch 1, gekennzeichnet durch Verwendung eines geformten Trägerkörpers als inertes Trägermaterial.

3. Verfahren nach Ansprüchen 1–2, dadurch gekennzeichnet, daß das oxidierbare Metall und/oder die Metallverbindung aus der Gruppe von Zinn, Blei, Mangan, Arsen, Antimon und Verbindungen derselben ausgewählt ist.

4. Verfahren nach Anspruch 3, gekennzeichnet durch Verwendung einer Sn(II)-Verbindung.

5. Verfahren nach Anspruch 4, gekennzeichnet durch Verwendung gegebenenfalls wasserhaltigen SnO.

6. Verfahren nach Ansprüchen 1–2, dadurch gekennzeichnet, daß das Trägermaterial, auf dem das

oxidierbare Metall oder die oxidierbare Metallverbindung vorhanden ist, hergestellt wurde durch Reduktion einer auf das Trägermaterial aufgebrachten reduzierbaren Metallverbindung.

7. Verfahren nach Anspruch 6, gekennzeichnet durch Verwendung einer Sn(IV)-Verbindung als reduzierbare Metallverbindung.

8. Verfahren nach Anspruch 7, gekennzeichnet durch Verwendung von kalziniertem $SiO_2$ und/oder $Al_2O_3$ als Trägermaterial.

9. Verfahren nach Ansprüchen 1–2, gekennzeichnet durch Verwendung von kalziniertem $SiO_2$ und/oder $Al_2O_3$ als Trägermaterial.

10. Verfahren nach Anspruch 9, gekennzeichnet durch Verwendung von $\alpha$-$Al_2O_3$.

11. Verfahren nach Ansprüchen 1–2, dadurch gekennzeichnet, daß die Reduktion der Silberionen in einer im wesentlichen sauerstofffreien Atmosphäre erfolgt.

## Revendications

1. Procédé de préparation d'un catalyseur à base d'argent sur un support inerte, qui comprend la précipitation d'ions argent à partir d'une solution par réduction en argent métallique en présence d'une matière support inerte, caractérisé en ce qu'on effectue la réduction principalement à l'aide d'un agent réducteur pour les ions argent, qui est présent à la surface de la matière inerte support sous forme d'un métal et/ou dérivé d'un métal oxydable(s).

2. Procédé selon la revendication 1, caractérisé en ce qu'on utilise, à titre de matière inerte support, un corps support façonné.

3. Procédé selon les revendications 1 et 2, caractérisé en ce que le métal et/ou le dérivé de métal oxydable(s) est (sont) choisi(s) parmi l'étain, le plomb, le manganèse, l'arsenic, l'antimoine et leurs dérivés.

4. Procédé selon la revendication 3, caractérisé en ce qu'on utilise un dérivé de Sn(II).

5. Procédé selon la revendication 4, caractérisé en ce qu'on utilise SnO éventuellement hydraté.

6. Procédé selon la revendication 1 et 2, caractérisé en ce que la matière support sur laquelle est présent le métal oxydable ou le dérivé de métal oxydable, a été produite par réduction d'un dérivé de métal réductible appliqué à la matière support.

7. Procédé selon la revendication 6, caractérisé en ce qu'on utilise comme dérivé de métal réductible un dérivé de Sn(IV).

8. Procédé selon la revendication 7, caractérisé en ce qu'on utilise comme matière support $SiO_2$ calciné et/ou $Al_2O_3$ calciné.

9. Procédé selon les revendications 1 et 2, caractérisé en ce qu'on utilise comme matière support $SiO_2$ calciné et/ou $Al_2O_3$ calciné.

10. Procédé selon la revendication 9, caractérisé en ce qu'on utilise l'alpha-$Al_2O_3$.

11. Procédé selon les revendications 1 et 2, caractérisé en ce qu'on effectue la réduction des ions argent dans une atmosphère pratiquement exempte d'oxygène.